# FASCICULE DE BREVET EUROPEEN

(11) **EP 3 225 244 B1**
(45) Date de publication et mention de la délivrance du brevet: **14.12.2022**
(21) Numéro de dépôt: 17163643.4
(22) Date de dépôt: 29.03.2017
(51) Int. Cl.: A61K 31/765, A61K 31/80, A61K 33/14, A61P 1/10

(54) **PREPARATIONS COLIQUES**
COLON ZUBEREITUNGEN
BOWEL PREPARATIONS

(30) Priorité: 29.03.2016 FR 1652693
(43) Date de publication de la demande: 04.10.2017
(73) Titulaire: CRITERIO, 3397 Roeser (LU)
(72) Inventeur: GIRARD, Marc, 38130 ECHIROLLES (FR)
(74) Mandataire: Lavoix

(56) Documents cités:
- EP-A1- 0 900 562
- EP-A1- 2 322 190
- WO-A1-2013/059881
- WO-A1-2014/040994
- WO-A2-2010/006209
- US-A1- 2015 056 307
- BUCCI C ET AL: "OC.01.8 SPLIT VS SAME-DAY REGIMES FOR BOWEL PREPARATION BEFORE COLONOSCOPY: A META-ANALYSIS OF PUBLISHED STUDIES", DIGESTIVE AND LIVER DISEASE, vol. 48, 27 février 2016 (2016-02-27), XP029429104, ISSN: 1590-8658, DOI: 10.1016/S1590-8658(16)30020-2

## Description

La présente invention concerne une composition colique comprenant notamment de la diméticone, utilisable lors des procédures diagnostiques, thérapeutiques ou chirurgicales sur l'intestin et plus particulièrement le côlon comme par exemple lors d'examens radiologiques, de la chirurgie digestive, ou des coloscopies.

La coloscopie est une technique d'examen exploratoire visuelle de la paroi interne du côlon. La coloscopie permet notamment de déceler des anomalies (polypes, ...) et d'effectuer des prélèvements. Le côlon doit être parfaitement propre pour permettre un examen précis.

Le patient doit suivre, quelques jours avant la coloscopie, un régime particulier et doit être à jeun le jour de l'examen. La veille de l'examen, le patient doit généralement ingérer, en moins d'une heure, deux fois deux litres d'une solution saline purgative, appelée préparation colique, provoquant des diarrhées et permettant de vider le côlon. L'efficacité des préparations coliques conditionne directement la qualité et les performances de l'examen. Le défaut de préparation est responsable de 40% des échecs de coloscopie (soit environ 6% des examens) (Aliment Pharmacol Their. 2007;25:373-4) et peut avoir des conséquences graves par la méconnaissance de lésions précancéreuses. Son impact économique est important, de l'ordre de 40 millions d'euros en France en 2014 (Acta Endosc.2015 ; 45 :211-6).

A ce jour, six familles de produits sont généralement utilisées : les préparations hyperosmotiques, les picosulfates de sodium, les sulfates de sodium, les macrogols pleines doses et les macrogols faibles volumes. Les phosphates de sodium (Fleet phosphosoda ^{©} - Colo Kit ^{©}) sont délaissés en raison des complications qu'ils peuvent entrainer même chez des sujets jeunes et en bonne santé. Les préparations hyperosmotiques (citrate de magnésium) agissent par mécanisme osmotique en entrainant un appel d'eau à travers la paroi intestinale. Ils sont bien tolérés et requièrent de faibles volumes de préparation, cependant ils restent peu utilisés en raison de leurs dangers sur la fonction rénale et les troubles électrolytiques qu'ils entrainent. Ils sont contre indiqués en cas de pathologie rénale, cardiaque, hépatique, et en cas d'anomalie des constantes biologiques sanguines. Les picosulfates de sodium (Picoprep ^{©}-Citrafleet ^{©}) représentent 23% des préparations coliques en France et ont comme principaux inconvénients un mauvais nettoyage du côlon droit, et de nombreuses contre-indications, notamment chez les personnes âgées, l'insuffisance rénale, les perturbations des valeurs biologiques sanguines, l'insuffisance cardiaque, l'insuffisance hépatique. La prudence est recommandée chez les patients sous diurétiques, anti-inflammatoires, corticoïdes, antidépresseurs et lithium. Ils présentent cependant une meilleure acceptabilité de la préparation. Les sulfates de sodium (Izinova ^{©}) ont comme principaux inconvénients un mauvais nettoyage du côlon droit, et de nombreuses contre-indications, notamment chez les personnes âgées de plus de 75 ans, l'insuffisance rénale, les perturbation des valeurs biologiques sanguines, l'insuffisance cardiaque, l'insuffisance hépatique ou encore la prise de certains antihypertenseurs couramment prescrits comme les diurétiques, les inhibiteurs de l'enzyme de conversion de l'angiotensine (IEC), antagonistes des récepteurs de l'angiotensine (ARA). La prise d'AINS est aussi contre indiquée. Les macrogols (Colopeg ^{©}, Fortran ^{©}, Klean prep ^{©} et Moviprep ^{©}) totalisent 57% des préparations coliques en France. Leur principal avantage par rapport aux molécules citées ci-avant est qu'ils ne souffrent pas de contre-indications et précautions d'emploi. Ce dernier point permet de simplifier les protocoles de prescription et évite de prescrire par erreur une préparation à un patient à risque de par son âge, une tare associée, une interaction médicamenteuse ou encore une grossesse. L'utilisation des polyethylenes glycols (PEG) a été proposée dès 1980 (Gastroenterology 1980 ; 78 :991-5). Les macrogols permettent un bon nettoyage de tous les segments du côlon. Cependant leur acceptabilité est médiocre. De manière générale, les préparations par macrogols nécessitent l'ingestion d'une quantité importante de liquide (quatre litres) dans un temps court. De plus, la prise de ces préparations coliques doit suivre un schéma particulier qui peut ne pas être compris par les patients dans 20% des cas. Enfin, le goût salé des préparations par PEG est généralement mal toléré. L'amélioration de la tolérance de la prise des Macrogols est un enjeu majeur. Plusieurs solutions ont été étudiées pour tenter de diminuer le volume de liquide à ingérer : pour les préparations dites de faibles volumes les PEG sont associées à de l'acide ascorbique et ascorbate de sodium (Glycoprep C Pharrnatel WO 89/05659 (Borody), Moviprep Norgine Pharma US 7.658,914 B2) ou de l'acide citrique et du citrate de sodium (Zanarotti Alessandro EP 2.322,190 A1). En raison de leur arôme agrume, elles doivent être absorbées glacées, de ce fait les patients sont tentés de les ingurgiter rapidement ce qui entraine des phénomènes de nausées et de vomissements. L'association de PEG et de laxatifs stimulants a été proposée notamment PEG et Bisocodyl^{®} (HalfLytely) mais son utilisation n'est pas recommandée en raison de cas de colites ischémiques graves (Am J Gastroenterol 2005; 100:2134.) et (Am J Gastroenterol 2010; 105:700.) PEG et Bisoxatin n'a pas été commercialisé (Borody Thomas Julius WO/2013/059981 A1). EP0900562 divulgue une composition de lavage pour l'intestin comprenant un composé soluble dans l'eau de haut poids moléculaire et un électrolyte.

Le vomissement est la manifestation d'un défaut de la vidange gastrique. Les vomissements sont décrits dans 16% des préparations coliques, et sont associés à une mauvaise préparation dans 40% des cas (Gastroenterol Clin Biol 2009;33:A180.). Pour accélérer la vidange gastrique, l'ajout de prokinétiques aux PEG a été étudiée : PEG et Métoclopramine^{®} (Primpéran) (Clin J Med 2010; 77:317.), mais son application est limitée en raison d'effets indésirables potentiellement graves (HAS Oct 2015). Boire la préparation avec une paille a été proposé, mais en pratique cette solution est difficilement compatible avec l'ingestion de grands volumes de liquides (quatre litres).

Les préparations actuellement disponibles recommandent une prise froide ou glacée afin d'en améliorer la tolérance, mais la prise de liquides glacés favorise les nausées et les vomissements en diminuant significativement la vitesse de vidange gastrique. (J Gastroenterol.2009;44(5) :412-8).

Il existe donc un intérêt à proposer une solution permettant de limiter, voire de supprimer, les nausées et vomissements.

La présence de mousse dans la lumière digestive lors de coloscopies est également connue. L'adjonction de siméticone à une préparation colique par PEG a été proposée dès 1988 (Dig Dis Sci 1988 ;33(2) :185-8). Elle améliorait la qualité de la préparation et surtout elle empêchait la formation de mousse dans le côlon.

Pendant la coloscopie, l'opérateur peut aspirer les liquides résiduels et laver le côlon à l'aide d'une pompe mécanique (Flushing pump ^{®}). Ce lavage mécanique complémentaire est pratiqué dans 100% des coloscopies. Cependant, cette procédure de lavage à l'eau sous faible pression fait apparaître une mousse abondante qui peut masquer jusqu'à 90% de la lumière digestive indépendamment de la présence de selles. Le phénomène peut donc apparaître sur un côlon parfaitement propre et masquer la muqueuse.

Indépendamment du volume de prise, l'optimisation de la préparation colique passe par une préparation à la carte. La préparation fractionnée (split dosing) avec la moitié de la dose prise la veille et la seconde moitié prise cinq à sept heures avant de l'examen permet d'obtenir une meilleure préparation que la prise totale du produit la veille. Plus on augmente le délai entre la fin de la prise de la préparation et le début de la coloscopie moins bonne est la préparation (Gastrointestinal Endoscopy 2010;72:313-20).

Il existe donc un intérêt à fournir des préparations coliques pouvant être prise de manière fractionnée pour améliorer leur acceptabilité.

Il existe également un intérêt à améliorer la présentation gustative des préparations coliques pour améliorer leur acceptabilité.

Il existe un intérêt à fournir une solution à la production de mousse abondante lors des lavages mécaniques du côlon.

D'autres objectifs encore apparaîtront à la lecture de la description de l'invention qui suit. Tout objet non décrit par les revendications ne fait pas partie de l'invention.

Il est décrit une composition déshydratée (A), notamment à reconstituer avec de l'eau, comprenant :
(a) au moins un composé susceptible de générer des contractions de l'intestin choisi parmi les sels de sodium, notamment sulfate de sodium, picosulfates de sodium, phosphates de sodium ; les sulfates de magnésium ; les sulfates de potassium ; les polyethylene glycol (PEG), seuls ou en mélange, de préférence PEG et sulfate de sodium ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ou siméticone, de préférence diméticone ;
(d) agents de saveurs.

Les agents de saveur sont des agents donnant un goût à la composition, les agents de saveur sont notamment choisis parmi des légumes, pomme de terre, fruits, épices, substitut de sucre, arômes, bouillon de viande (notamment de volaille), extraits de plantes (notamment extrait de menthe, camomille, verveine, oranger et/ou tilleul), seuls ou en mélange.

La composition peut également comprendre du citrate de sodium.

De préférence, la composition est exempte de citrate de sodium.

La diméticone et la siméticone appartiennent à la classe des polydimethylsiloxane linéaires et ont pour formule générale :

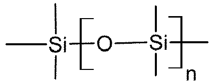

De façon avantageuse, l'utilisation de la diméticone ou de la siméticone dans les préparations coliques permet d'éviter la formation de mousse dans le côlon ou d'entraîner une coalescence extrêmement rapide des bulles si celles-ci se forment.

Les agents osmotiques, notamment chlorure de sodium, chlorure de potassium et bicarbonate de sodium, sont importants pour assurer une osmolarité physiologique de la préparation et éviter une absorption ou des fuites d'électrolytes à travers la paroi intestinale.

Les objectifs précités sont remplis par la présente invention qui propose une composition déshydratée (A), à reconstituer avec de l'eau et à prendre chaude à 60°C, comprenant un polyéthylène glycol (PEG) (notamment PEG 3500 ou PEG 4000), du sulfate de sodium, du chlorure de sodium, du chlorure de potassium, du bicarbonate de sodium, de la diméticone et des agents de saveurs tels que définis ci-dessus, pour son utilisation pour induire une élimination des selles lors de procédures diagnostique, thérapeutique ou chirurgicales sur l'intestin. La composition peut en outre comprendre du citrate de sodium. De préférence, la composition de l'invention est exempte de citrate de sodium.

La présente invention concerne également une composition déshydratée (A), à reconstituer avec de l'eau et à prendre chaude à 60°C, comprenant :
(a) de 60 à 90 % en poids de PEG ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone;
(d) agents de saveurs choisis parmi des légumes, pomme de terre, épices, substitut de sucre, arômes, bouillon de viande (notamment de volaille), extraits de plantes (notamment extrait de menthe, camomille, verveine, oranger et/ou tilleul), seuls ou en mélange,
pour son utilisation pour induire une élimination des selles lors de procédures diagnostique, thérapeutique ou chirurgicales sur l'intestin.

De façon avantageuse, la reconstitution de la préparation avec de l'eau permet d'obtenir une préparation pour le nettoyage de l'intestin.

La présente invention concerne donc également une telle composition déshydratée pour son utilisation pour le lavage du colon au préalable d'un examen exploratoire du côlon, par exemple coloscopie.

La composition (A) de la présente invention se présente sous forme de poudre, granulés ou toutes autres formes adaptées comme composition déshydratée et susceptible d'être reconstituée par ajout d'eau. La présentation en poudre est préférée.

Le conditionnement préférentiel de la composition (A) sous forme de sachet unitaire.

L'osmolarité de la solution reconstituée est comprise entre 190-600 mOsmol/l et préférentiellement entre 240 et 250 mOsmol/l.

La teneur en électrolytes de la solution reconstituée est telle que l'on peut considérer comme nul les échanges électrolytes intestin plasma.

Le pH de la solution reconstituée est compris entre 3,9 et 5,0 et préférentiellement entre 4,3 et 4,8.

La composition (A) comprend de 0,3 à 5 g de diméticone. La composition reconstituée comprend 1,3 à 5 g de diméticone par litre de composition.

Préférentiellement le composé susceptible de générer des contractions de l'intestin, de préférence PEG, est compris entre 30 et 130 g dans la composition (A).

Le sulfate de sodium est compris entre 0 et 10 g dans la composition (A).

La quantité d'agent osmotique favorisant la rétention d'eau et la pression osmotique est comprise entre 1et 10 g dans la composition (A). De préférence, l'agent osmotique est un mélange de chlorure de sodium, de chlorure de potassium et de bicarbonate de sodium, le chlorure de sodium est compris entre 1 et 2,5 g, le chlorure de potassium est compris entre 0,2 et 1,5 g, le bicarbonate de sodium est compris entre 0 et 3 g.

De préférence la composition (A) comprend de 1 à 3 % en poids de diméticone.

De préférence, la composition (A) comprend de 60 à 90 % en poids de composé susceptible de générer des contractions de l'intestin, de préférence PEG.

De préférence, la composition (A) comprend de 7 à 9 % en poids de sulfate de sodium.

De préférence, la composition (A) comprend de 9 à 15 % en poids d'agent osmotique.

De préférence, la composition (A) comprend de 20 à 60 % en poids d'agents de saveur. Le polyéthylène glycol de la composition (A) peut notamment être choisi parmi les polyéthylènes glycol typiquement mis en œuvre dans les préparations coliques. De préférence, le polyéthylène glycol est choisi parmi les polyéthylène glycol présentant un MW supérieur à 2000 g/mol, de préférence supérieur à 2500 g/mol, de préférence inférieur à 4500 g/mol, de préférence le polyéthylène glycol (PEG) est un PEG 3500 ou PEG 4000 (présentant respectivement un MW de 3500 g/mol et de 4000 g/mol), typiquement connu sous le nom Macrogol.

Après reconstitution avec de l'eau (typiquement ajout de 0,5 à 2 litres d'eau), la composition (A) permet l'obtention d'une préparation colique qui peut notamment être sous forme de repas liquides froids ou chauds, de préférence chauds, et comprenant de la diméticone Il est également décrit un nouveau plan d'administration de ces préparations coliques.

L'administration de la préparation reconstituée va induire une élimination des selles recherchée lors des procédures diagnostiques, thérapeutiques ou chirurgicales sur l'intestin et plus particulièrement le côlon comme par exemple lors d'examens radiologiques, de la chirurgie digestive, ou des coloscopies.

Les agents de saveurs inclus dans la formulation ont pour but premier de permettre la consommation chaude de ladite solution, mais aussi d'améliorer le goût, et ainsi de favoriser la tolérance et l'acceptabilité de la prise des préparations coliques par les patients.

Les préparations conventionnelles les plus couramment prescrites, par PEG (Colopeg^{®}) et les PEG dits faibles volumes car associés à de l'acide ascorbique et ascorbate de sodium (Moviprep^{®}), recommandent une préparation avec les liquides froids ou de conserver la préparation au frais avant utilisation. La vitesse de vidange gastrique précoce est significativement supérieure pour l'ingestion de liquides à 60°C comparée aux liquides froids. La prise de liquides chauds accélère la vitesse de vidange gastrique (J Gastroenterol.2009 ;44(5) :412-8). Une vitesse de vidange gastrique élevée diminue les phénomènes de dyspepsie comme les nausées. Un arrêt ou un ralentissement majeur de la vidange gastrique entraine des vomissements. Les nausées et les vomissements sont retrouvés dans 8 à 25 % des préparations coliques (Gut; 210:P0220).

Les inventeurs ont montré de façon surprenante que la prise de la présente formulation était aussi efficace et mieux tolérée que les préparations existantes. Trois litres de la composition (A) reconstituée prise à 60°C ont été comparés à quatre litres froids de PEG (Colopeg^{®}) avec la même efficacité et une tolérance accrue. Trois litres de la composition (A) reconstituée prise à 60°C ont été comparés à deux litres froids de PEG dit faible volume car avec une association d'acide ascorbique, ascorbate de sodium (Moviprep^{®}). L'efficacité de la composition selon l'invention a été supérieure et mieux tolérée que la préparation avec PEG dit faible volume.

Il a été découvert de façon surprenante que la composition chaude avec agents de saveur, était mieux acceptée et mieux tolérée, alors que le volume total à ingérer pouvait être supérieur de 50% aux préparations classiques, et que les patients mettaient 25% de temps supplémentaire pour l'absorber. Par ailleurs, les inventeurs ont demandé à des patients de choisir entre les préparations classiques à base de PEG froides ou chaudes et les patients ont tous préférés prendre la préparation froide du fait du mauvais goût des préparations PEG classiques. L'association d'un PEG avec de l'acide citrique ou de l'acide ascorbique et ascorbate de sodium (Moviprep), permet de réduire la quantité de liquide à ingérer mais oblige à boire le mélange froid ou à conserver la préparation au frais avant utilisation. Les inventeurs ont montré que les patients avaient tendance à boire rapidement les préparations froides ce qui amenait des nausées et vomissements.

La prise de tout ou partie de la composition selon l'invention sous forme d'une solution chaude, notamment sous la forme de repas liquide, entraine une modification du comportement des patients avec une absorption significativement plus lente de la solution améliorant ainsi son efficacité et sa tolérance.

De ce fait la prise de la composition selon l'invention évite les phénomènes de vomissements constatés dans 8 à 25 % des cas avec les préparations conventionnelles.

Dans une étude clinique portant sur des volontaires sains les inventeurs ont démontré que la prise chaude à 60° de préparations coliques conventionnelles par PEG (Colopeg^{®}) était impossible au-delà de un litre, alors que quatre litres sont requis et que des nausées ou vomissements apparaissaient dans 30% des cas. Les inventeurs ont démontré que la prise chaude à 60°C de PEG dits faibles volumes car associés à de l'acide ascorbique et ascorbate de sodium (Moviprep^{®}) était impossible au-delà de un litre, alors que deux litres sont requis et que des nausées ou vomissements apparaissaient dans 60% des cas.

Ainsi les inventeurs ont montré que la composition prise selon l'invention induit un effet laxatif identique ou supérieur aux préparations conventionnelles avec une tolérance et une acceptabilité accrue de la part des patients.

Il est décrit une composition déshydratée (A1) comprenant :
(a) au moins un composé susceptible de générer des contractions de l'intestin choisi parmi les sels de sodium, notamment sulfate de sodium, picosulfates de sodium, phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrates de magnésium seuls ou en mélange ;
(c) diméticone ou siméticone, de préférence diméticone ;
(d) au moins un élément choisi parmi :
   - de la fécule de pomme de terre,
   - des arômes,
   - du bouillon, par exemple bouillon de volaille, de préférence bouillon de poule,
   - des légumes,
   - des épices,
   - du jus d'oignon,
   - seuls ou en mélange.

Cette préparation (A1) peut être prise chaude sous forme d'entrée ou de plat dans l'optique d'une administration entrée/plat/dessert/boisson.

La composition (A1) peut également comprendre du citrate de sodium.

De préférence, la composition (A1) est exempte de citrate de sodium.

Il est décrit une préparation (A2) comprenant :
(a) au moins un composé susceptible de générer des contractions de l'intestin choisi parmi les sels de sodium, notamment sulfate de sodium, picosulfates de sodium, phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ou siméticone, de préférence diméticone ;
(d) au moins un élément choisi parmi :
   - un ou plusieurs fruits,
   - des épices,
   - substitut de sucre,
   - seuls ou en mélange.

Cette préparation (A2) peut être prise froide sous forme de dessert dans l'optique d'une administration entrée/plat/dessert/boisson.

La composition (A2) peut en outre comprendre du citrate de sodium.

De préférence, la composition (A2) est exempte de citrate de sodium.

Il est décrit une préparation déshydratée (A3) comprenant :
(a) au moins un composé susceptible de générer des contractions de l'intestin choisi parmi les sels de sodium, notamment sulfate de sodium, picosulfates de sodium, phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ou siméticone, de préférence diméticone ;
(d) au moins un élément choisi parmi :
   - des extraits de plantes,
   - des arômes,
   - substitut de sucre,
   - seuls ou en mélange.

Cette préparation (A3) peut être prise chaude sous forme d'une boisson dans l'optique d'une administration entrée/plat/dessert/boisson.

La composition (A3) peut en outre comprendre du citrate de sodium.

De préférence, la composition (A3) est exempte de citrate de sodium.

On entend par « substitut de sucre » désigner notamment les édulcorants, de façon avantageuse, les substituts de sucre sont choisis parmi saccharine, cyclamate, aspartame, acésulfame-K, thaumatine, sucralose, stévia, polyalcools et polydextrose, de préférence stévia.

De façon avantageuse, la mise en forme des préparations coliques décrites autorise la consommation à 60°C et permet d'améliorer le goût des dites préparations et donc l'acceptabilité par le patient. De plus, ces compositions doivent être prises de façons fractionnées comme un repas classique et chaudes à 60°C, augmentant également l'acceptabilité par le patient.

Ainsi, il est possible de prendre :
- la composition (A1) comme entrée et/ou plat lors d'un repas,
- la composition (A2) comme un dessert lors d'un repas, et
- la composition (A3) comme une boisson chaude.

Il est décrit un kit comprenant :
- au moins une première composition (A) selon l'invention comprenant au moins un légume ; et
- au moins une deuxième composition (A) selon l'invention comprenant au moins un fruit ;
Notamment pour son utilisation séquencée, notamment comme préparation colique, pour le lavage du côlon.

Il est décrit un kit comprenant :
- au moins une première composition (A) selon l'invention comprenant au moins un légume ; et
- au moins une deuxième composition (A) selon l'invention comprenant au moins un extrait de plantes ou arôme ;
notamment pour son utilisation séquencée, notamment comme préparation colique, pour le lavage du côlon.

Il est décrit un kit comprenant :
- au moins une première composition (A) selon l'invention comprenant au moins un fruit ; et
- au moins une deuxième composition (A) selon l'invention comprenant au moins un extrait de plantes ou arôme ;
notamment pour son utilisation séquencée, notamment comme préparation colique, pour le lavage du côlon.

Il est décrit un kit comprenant :
- au moins une première composition (A) selon l'invention comprenant au moins un légume ; et
- au moins une deuxième composition (A) selon l'invention comprenant au moins un fruit ; et
- au moins une troisième composition (A) selon l'invention comprenant au moins un extrait de plantes ou arôme ;
notamment pour son utilisation séquencée, notamment comme préparation colique, pour le lavage du côlon.

La présente invention concerne également un kit comprenant :
- au moins deux compositions déshydratées à reconstituer avec de l'eau et à prendre chaude à 60°C comprenant :
   (a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
   (b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
   (c) diméticone ;
   (d) au moins un légume; et
- au moins une composition déshydratée à reconstituer avec de l'eau comprenant :
   (a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
   (b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
   (c) diméticone ;
   (d) au moins un fruit, et
- au moins une composition déshydratée à reconstituer avec de l'eau et à prendre chaude à 60°C comprenant :
   (a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
   (b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
   (c) diméticone ;
   (d) au moins un extrait de plantes,
pour son utilisation séquencée pour induire une élimination des selles préalable à la coloscopie.

De préférence, le kit comprend :
- deux compositions déshydratées à reconstituer avec de l'eau et à prendre chaudes à 60°C comprenant :
   (a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
   (b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
   (c) diméticone ;
   (d) au moins un légume; et
- une composition deshydratée à reconstituer avec de l'eau comprenant :
   (a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
   (b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
   (c) diméticone ;
   (d) au moins un fruit, et
- deux compositions deshydratées à reconstituer avec de l'eau et à prendre chaudes à 60°C comprenant :
   (a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
   (b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
   (c) diméticone ;
   (d) au moins un extrait de plante ou arôme.

De préférence dans le kit les compositions comprennent entre 1 et 3% en poids de diméthicone.

De préférence, dans le kit les compositions sont exemptes de citrate de sodium.

De préférence, le kit comprend une notice précisant que le dîner, la veille de la coloscopie, doit être remplacé par la prise d'une composition comprenant au moins un légume comme entrée, de la deuxième composition comprenant au moins un légume comme plat, de la composition comprenant au moins un fruit comme dessert et d'une composition comprenant au moins un extrait de plantes ou arôme comme boisson chaude ou froide, de préférence chaude, et la prise, le jour de la coloscopie de la deuxième composition comprenant au moins un extrait de plantes ou arôme comme boisson chaude ou froide, de préférence chaude.

Il est décrit un kit comprenant :
- deux compositions (A1) selon l'invention ;
- une composition (A2) selon l'invention ;
- deux compositions (A3) selon l'invention ;
notamment pour leur utilisation séquencée, notamment comme préparation colique, pour le lavage du côlon.

De préférence, le kit comprend une notice précisant que le dîner, la veille de la coloscopie, doit être remplacé par la prise d'une composition (A1) comprenant au moins un légume comme entrée, de la deuxième composition (A1) comprenant au moins un légume comme plat, de la composition (A2) comprenant au moins un fruit comme dessert et d'une composition (A3) comprenant au moins un extrait de plantes ou arôme comme boisson chaude ou froide, et la prise, le jour de la coloscopie de la deuxième composition (A3) comprenant au moins un extrait de plantes ou arôme comme boisson chaude ou froide.

Il est décrit que les compositions (A1), (A2) et (A3), peuvent être utilisées comme préparation colique en vue d'un examen coloscopique. Ainsi, il est décrit un régime, préalable à la coloscopie, comprenant les étapes suivantes :
- la veille au soir de la coloscopie, remplacer son dîner par la prise :
   ∘ d'une composition selon l'invention comprenant des légumes, notamment la prise d'une composition (A1), comme entrée ; et/ou
   ∘ d'une composition selon l'invention comprenant des légumes, notamment la prise d'une composition (A1), comme plat ; et/ou
   ∘ d'une composition selon l'invention comprenant des fruits, notamment la prise d'une composition (A2), comme dessert ; et/ou
   ∘ d'une composition selon l'invention comprenant un extrait de plantes ou des arômes, notamment la prise d'une composition (A3), comme boisson chaude ou froide, de préférence chaude ; et/ou
- le jour de la coloscopie, de préférence au moins 6 heure avant, la prise d'une composition selon l'invention comprenant un extrait de plantes ou des arômes, notamment la prise d'une composition (A3), comme boisson chaude à 60°C de préférence ou froide.

De préférence, le régime préalable à la coloscopie, comprenant les étapes suivantes :
- la veille au soir de la coloscopie, remplacer son dîner par la prise :
   ∘ d'une première composition (A1), comme entrée ;
   ∘ d'une seconde composition (A1), comme plat ;
   ∘ d'une composition (A2), comme dessert ;
   ∘ d'une première composition (A3), comme boisson chaude ou froide, de préférence chaude ; et
- le jour de la coloscopie, de préférence au moins 6 heures avant, la prise d'une seconde composition (A3), comme boisson chaude ou froide, de préférence chaude.

Un exemple de composition (A1) pouvant être prise chaude à 60°C comme une entrée lors d'un repas est notamment la composition suivante :
250 ml d'eau, 13,5 g de macrogol 3350, 1,3 g de sulfate de sodium anhydre, 0,335 g de chlorure de sodium, 0,17 g de chlorure de potassium, 0,38 g de bicarbonate de sodium, 0,343 g de diméticone, et 5 g d'une composition comprenant : fécule de pomme de terre, arômes, graisse de poule, arôme de romarin, légumes 1,1% (oignon, carottes, céleri) épice, aromates, curcuma, jus d'oignon.

Un exemple de composition (A1) pouvant être prise chaude à 60°C comme un plat lors d'un repas est notamment la composition suivante :
1 litre d'eau, 54 g de macrogol 3350, 5,2 g de sulfate de sodium anhydre, 1,34 g de chlorure de sodium, 0,68 g de chlorure de potassium, 1,54 g de bicarbonate de sodium, 1,37 g de diméticone et 33 g d'une composition comprenant : fécule de pomme de terre, légumes 2% (soja, carottes, oignon, poireau, céleri), graisse de poule, arômes blé et soja, arôme de champignons noirs, arôme coco, arôme de romarin, épices : curry, poivre, moutarde, curcuma, coriandre, cannelle.

Un exemple de composition (A2) pouvant être prise à 20° comme un dessert lors d'un repas est notamment la composition suivante :
7,55 g de macrogol 3350, 0,72 g de sulfate de sodium anhydre, 0,19 g de chlorure de sodium, 0,1 g de chlorure de potassium, 0,22g de bicarbonate de sodium, 0,19 g de diméticone et 120 g d'une composition comprenant : compote de pomme 70%, banane 30%, cannelle, stévia, coulis de framboise.

Un exemple de composition (A3) pouvant être prise comme boisson chaude de préférence à 60°C mais aussi froide lors d'un repas par exemple, est notamment la composition suivante :
250 ml d'eau, 13,5 g de Macrogol 3350, 1,3 g de sulfate de sodium anhydre, 0,335 g de chlorure de sodium, 0,17 g de chlorure de potassium, 0,30 g de bicarbonate de sodium, 0,25 g d'acide ascorbique, 0,25 g d'ascorbate de sodium, 0,343 g de diméticone et 10 g d'un mélange comprenant : stévia, extraits de plantes 2,4% (menthe, camomille, verveine, oranger, tilleul), arôme de menthe, arôme de thé noir.

Un autre exemple de composition (A3) pouvent être prise comme boisson chaude de préférence à 60°C mais aussi froide lors d'un repas par exemple, est notamment la composition suivante :
500 ml d'eau, 29,5 g de Macrogol 3350, 2.84 g de sulfate de sodium anhydre, 0,73 g de chlorure de sodium, 0,37 g de chlorure de potassium, 0,84 g de bicarbonate de sodium, 2,25 g de diméticone et 20 g d'un mélange comprenant : 0,095 g d'aspartam, extraits de plantes 2,4% (menthe, camomille, verveine, oranger, tilleul), arome de menthe, arome de thé noir.

Un autre exemple de composition (A3) pouvant être prise comme boisson chaude de préférence à 60°C mais aussi éventuellement froide le jour de l'examen, six heures avant l'anesthésie est notamment la composition suivante :
1,5 litre d'eau, 88,5 g de Macrogol 3350, 8,525 g de sulfate de sodium anhydre, 2,199 g de chlorure de sodium, 1,1162 g de chlorure de potassium, 1,1 g de bicarbonate de sodium, 1 g d'acide ascorbique, 1g d'ascorbate de sodium, 4,498 g de diméticone et 60 g d'un mélange comprenant stévia, extrait de plantes 2,4%(menthe, camomille, verveine, oranger, tilleul), arôme de menthe, arôme de thé noir.

Un autre exemple de composition (A3) pouvant être prise comme boisson chaude de préférence à 60°C mais aussi éventuellement froide le jour de l'examen, six heures avant l'anesthésie est notamment la composition suivante :
1,5 litre d'eau, 88,5 g de Macrogol 3350, 8,525 g de sulfate de sodium anhydre, 2,199 g de chlorure de sodium, 1,1162 g de chlorure de potassium, 2.52 g de bicarbonate de sodium, 4,498 g de diméticone et 60 g d'un mélange comprenant 0.85 g d'aspartam, extrait de plantes 2,4%(menthe, camomille, verveine, oranger, tilleul), arome de menthe, arôme de thé noir.

Il est décrit un kit comprenant au moins une composition (A) et comprenant au moins une composition déshydratée (B), à reconstituer avec de l'eau, comprenant :
- au moins un composé susceptible de générer des contractions de l'intestin choisi parmi les sels de sodium, notamment sulfate de sodium, picosulfates de sodium, phosphates de sodium ; les sulfates de magnésium ; les sulfates de potassium ; les polyéthylène glycol (PEG), seuls ou en mélange ;
- au moins un agent osmotique favorisant la rétention d'eau et la pression osmotique dans le côlon, par exemple chlorure de sodium, chlorure de potassium, bicarbonate de sodium, citrate de magnésium seuls ou en mélange ;
- agents de saveurs choisis parmi des légumes, pomme de terre, fruits, épices, substitut de sucre, arômes, bouillon de viande (notamment de volaille), extraits de plantes (notamment extrait de menthe, camomille, verveine, oranger et/ou tilleul), seuls ou en mélange.

Notamment pour son utilisation séquencée, notamment comme préparation colique, pour le lavage du côlon.

Il est décrit un kit comprenant :
- au moins une première composition (B) comprenant au moins un légume ; et
- au moins une deuxième composition (A) comprenant au moins un fruit ;
Notamment pour son utilisation séquencée, notamment comme préparation colique, pour le lavage du côlon.

Il est décrit un kit comprenant :
- au moins une première composition (B) comprenant au moins un légume ; et
- au moins une deuxième composition (A) comprenant au moins un extrait de plantes ou arôme ;
notamment pour son utilisation séquencée, notamment comme préparation colique, pour le lavage du côlon.

Il est décrit un kit comprenant :
- au moins une première composition (B) comprenant au moins un légume ; et
- au moins une deuxième composition (A) comprenant au moins un fruit ; et
- au moins une troisième composition (A) comprenant au moins un extrait de plantes ou arôme ;
notamment pour son utilisation séquencée, notamment comme préparation colique, pour le lavage du côlon.

Il est décrit un kit comprenant :
- deux compositions (B) comprenant au moins un légume ; et
- une composition (A) comprenant au moins un fruit ; et
- deux compositions (A) comprenant au moins un extrait de plantes ou arôme ;
notamment pour son utilisation séquencée, notamment comme préparation colique, pour le lavage du côlon.

De préférence, le kit comprend une notice précisant que le dîner, la veille de la coloscopie, doit être remplacé par la prise d'une composition (B) comprenant au moins un légume comme entrée, de la deuxième composition (B) comprenant au moins un légume comme plat, de la composition (A) selon l'invention comprenant au moins un fruit comme dessert et d'une composition (A) selon l'invention comprenant au moins un extrait de plantes ou arôme comme boisson chaude ou froide, de préférence chaude, et la prise, le jour de la coloscopie de la deuxième composition (A) selon l'invention comprenant au moins un extrait de plantes ou arôme comme boisson chaude ou froide, de préférence chaude.

Il est décrit un kit comprenant :
- deux compositions (B);
- une composition (A2);
- deux compositions (A3);
notamment pour leur utilisation séquencée, notamment comme préparation colique, pour le lavage du côlon.

De préférence, le kit comprend une notice précisant que le dîner, la veille de la coloscopie, doit être remplacé par la prise d'une composition (B) comprenant au moins un légume comme entrée, de la deuxième composition (B) comprenant au moins un légume comme plat, de la composition (A2) comprenant au moins un fruit comme dessert et d'une composition (A3) comprenant au moins un extrait de plantes ou arôme comme boisson chaude ou froide, et la prise, le jour de la coloscopie de la deuxième composition (A3) comprenant au moins un extrait de plantes ou arôme comme boisson chaude ou froide.

De façon particulièrement avantageuse, les compositions (B) et (A) peuvent être utilisées comme préparation colique en vue d'un examen coloscopique. Ainsi, la présente invention propose un régime, préalable à la coloscopie, comprenant les étapes suivantes :
- la veille au soir de la coloscopie, remplacer son dîner par la prise :
   ∘ d'une composition (B) selon l'invention comprenant des légumes, comme entrée ; et/ou
   ∘ d'une composition (B) comprenant des légumes, comme plat ; et/ou
   ∘ d'une composition selon l'invention comprenant des fruits, notamment la prise d'une composition (A2), comme dessert ; et/ou
   ∘ d'une composition selon l'invention comprenant un extrait de plantes ou des arômes, notamment la prise d'une composition (A3), comme boisson chaude ou froide, de préférence chaude ; et/ou
- le jour de la coloscopie, de préférence au moins 6 heure avant, la prise d'une composition selon l'invention comprenant un extrait de plantes ou des arômes, notamment la prise d'une composition (A3), comme boisson chaude à 60°C de préférence ou froide.

De préférence, le régime préalable à la coloscopie, comprenant les étapes suivantes :
- la veille au soir de la coloscopie, remplacer son dîner par la prise :
   ∘ d'une première composition (B), comme entrée ;
   ∘ d'une seconde composition (B), comme plat ;
   ∘ d'une composition (A2), comme dessert ;
   ∘ d'une première composition (A3), comme boisson chaude ou froide, de préférence chaude ; et
- le jour de la coloscopie, de préférence au moins 6 heures avant, la prise d'une seconde composition (A3), comme boisson chaude ou froide, de préférence chaude.

Un exemple de composition (B) pouvant être prise chaude à 60°C comme une entrée lors d'un repas est notamment la composition suivante :
- 500 ml d'eau, 29,5 g de macrogol 3350, 2,841 g de sulfate de sodium anhydre, 0,73 g de chlorure de sodium, 0,373 g de chlorure de potassium, 0,84 g de bicarbonate de sodium, et 10 g d'une composition comprenant : fécule de pomme de terre, arômes, graisse de poule, arôme de romarin, légumes 1,1% (oignon, carottes, céleri) épice, aromates, curcuma, jus d'oignon.

Un exemple de composition (B) pouvant être prise chaude à 60°C comme un plat lors d'un repas est notamment la composition suivante :
1 litre d'eau, 59 g de macrogol 3350, 5,68 g de sulfate de sodium anhydre, 1,46 g de chlorure de sodium, 0,74 g de chlorure de potassium, 1,68 g de bicarbonate de sodium, et 33 g d'une composition comprenant : fécule de pomme de terre, légumes 2% (soja, carottes, oignon, poireau, céleri), graisse de poule, aromes blé et soja, arome de champignons noirs, arome coco, arome de romarin, épices : curry, poivre, moutarde, curcuma, coriandre, cannelle.

L'objectif de l'invention est d'améliorer la vidange gastrique tout en évitant les nausées et vomissements. Pour cela, les inventeurs ont montré de façon avantageuse l'importance : Du choix d'agents de saveurs permettant une prise à 60°C de la solution.

De préférence du choix de la prise à 60°C de la totalité des 3,5l de préparation.

De préférence, la présentation sous forme d'entrée, plat, dessert et infusion qui permet de modifier le comportement du patient, avec une prise beaucoup plus lente et donc mieux tolérée du produit, sans avoir recours aux artifices exposés ci avant (ingestion à l'aide d'une paille, association de PEG et métoclopramine, Bisacodyl, Bisoxatin, acide ascorbique et ascorbate de sodium ou acide citrique et citrate de sodium.)

Cette nouvelle approche originale de la préparation colique sous forme de repas, améliore la vitesse de vidange gastrique et permet une diminution significative des nausées et des vomissements par comparaison aux produits actuellement proposés. La présentation de l'invention sous forme de repas complet permet une meilleure compréhension de la procédure, contre 25 % de mésusage avec les procédures actuellement disponibles (étude observationnelle portant sur 1000 patients, réalisée à la demande de l'Agence Nationale de Sécurité du Médicament)

Dans le cadre de la présente invention, on entend par préparations coliques des compositions purgatives provoquant des diarrhées. L'objectif de ces préparations coliques est de vider le côlon.

Dans le cadre de la présente invention, on entend par liquide de lavage mécanique, une composition liquide (CL) généralement aqueuse utilisée pour laver le côlon et aspirer les éventuels liquides résiduels. Ce liquide de lavage mécanique est projeté dans le côlon à l'aide d'une pompe mécanique (Flushing pump^{©} Olympus). De telles compositions liquides de lavage mécanique sont connues de l'homme du métier, notamment la solution Colocleaner ^{©}.

Il est décrit une composition (CL) comprenant de l'eau et de la diméticone ou siméthicone pour son utilisation pour le lavage mécanique du côlon.

De préférence, la composition (CL) est utilisée pour le lavage mécanique du côlon notamment à l'aide d'une pompe, par exemple flushing pump, ou en lavement préalablement à la coloscopie. De préférence, la composition comprend également au moins un colorant, de préférence un colorant rouge. En effet, la présence d'un colorant permet au praticien de savoir que la composition de lavage est bien présente dans le réservoir de la pompe, de plus les colorants rouges n'altèrent pas la vision endoscopique et les performances de l'examen. De façon préférée, le colorant est choisi parmi le colorant rouge cochenille (E124), le colorant azorubine (E122) ou leur mélange.

De façon préférée dans cette composition (CL), la quantité de diméticone ou siméticone, de préférence diméticone, est comprise entre de 0,625 g à 4,5 g, de préférence de 1,125 g à 2,25 g par litre de composition.

De façon avantageuse, la composition (CL) pour son utilisation pour le lavage mécanique du côlon est une solution de Colocleaner^{©} à laquelle est ajoutée de la diméticone ou siméticone, de préférence diméticone, de préférence dans une concentration de 0,625 g à 4,5 g, de préférence de 1,125 g à 2,25 g par litre de composition.

Comme exemple de telle composition (CL) on peut citer la composition suivante :

| |
|---|
| Dimeticone 2,349 g dissout dans 15 cc d'eau stérile. |
| Colorant rouge cochenille (E 124) |
| Azorubine (E 122) |

Comme autre exemple de telle composition (CL) on peut citer la composition suivante :

| |
|---|
| Dimeticone 4 g dissout dans 15 cc d'eau stérile. |
| Colorant rouge cochenille (E 124) |
| Azorubine (E 122) |

Ainsi, la présente invention couvre également les kits pour leur utilisation tels que décrits ci-dessus comprenant en outre au moins une composition aqueuse notamment pour le lavage mécanique du côlon comprenant de la diméticone.

Il est également décrit une méthode de lavage du côlon ; notamment en vue d'une coloscopie, comprenant les étapes de :
- ingérer, par le patient, la veille au soir de l'examen, en remplacement du dîner :
   ∘ une première composition (A) prise chaude à 60° et comprenant au moins un légume, de préférence une première composition (A1), notamment comme entrée chaude ;
   ∘ une deuxième (A) prise chaude à 60° et comprenant au moins un légume, de préférence une deuxième composition (A1), notamment comme plat chaud ;
   ∘ une composition (A) prise à 20° ou froide et comprenant au moins un fruit, de préférence une composition (A2), notamment comme dessert ; et
   ∘ une composition (A) prise chaude à 60° et comprenant au moins un extrait de plantes ou arôme, de préférence une première composition (A3), notamment comme boisson de préférence chaude ou éventuellement froide,
   ∘ ingérer le jour de l'examen (jour de la coloscopie), de préférence 6 heures avant l'examen, une deuxième composition (A) prise chaude à 60° et comprenant au moins un extrait de plantes ou arôme, de préférence une deuxième composition (A3), notamment comme boisson de préférence chaude ou éventuellement froide,
- optionnellement, avant ou éventuellement pendant l'examen lavage mécanique du côlon par une composition aqueuse comprenant de la diméticone ou de la siméticone, de préférence de la diméticone.

Il est également décrit une méthode de préparation à la coloscopie, comprenant les étapes de :
- ingérer, par le patient, la veille au soir de l'examen, en remplacement du dîner :
   ∘ une première composition (A) prise chaude à 60° et comprenant au moins un légume, de préférence une première composition (A1), notamment comme entrée chaude;
   ∘ une deuxième (A) prise chaude à 60° et comprenant au moins un légume, de préférence une deuxième composition (A1), notamment comme plat chaud;
   ∘ une composition (A) prise à 20° ou froide comprenant au moins un fruit, de préférence une composition (A2), notamment comme dessert ; et
   ∘ une composition (A) prise chaude à 60° et comprenant au moins un extrait de plantes ou arôme, de préférence une première composition (A3), notamment comme boisson de préférence chaude ou éventuellement froide,
   ∘ ingérer le jour de l'examen (jour de la coloscopie), de préférence 6 heures avant l'examen, une deuxième composition (A) prise chaude à 60° et comprenant au moins un extrait de plantes ou arôme, de préférence une deuxième composition (A3), notamment comme boisson de préférence chaude ou éventuellement froide,
- optionnellement, avant ou éventuellement pendant l'examen, lavage mécanique du côlon par une composition aqueuse comprenant de la diméticone ou de la siméticone, de préférence de la diméticone.

Il est également décrit une méthode de lavage du côlon ; notamment en vue d'une coloscopie, comprenant les étapes de :
- ingérer, par le patient, la veille au soir de l'examen, en remplacement du dîner :
   ∘ une première composition (B) prise chaude à 60°C et comprenant au moins un légume, notamment comme entrée chaude ;
   ∘ une deuxième composition (B) prise chaude à 60°C et comprenant au moins un légume, notamment comme plat chaud ;
   ∘ une composition (A) prise à 20°C ou froide et comprenant au moins un fruit, de préférence une composition (A2), notamment comme dessert ; et
   ∘ une composition (A) prise chaude à 60°C et comprenant au moins un extrait de plantes ou arôme, de préférence une première composition (A3), notamment comme boisson de préférence chaude ou éventuellement froide,
   ∘ ingérer le jour de l'examen (jour de la coloscopie), de préférence 6 heures avant l'examen, une deuxième composition (A) prise chaude à 60°C et comprenant au moins un extrait de plantes ou arôme, de préférence une deuxième composition (A3), notamment comme boisson de préférence chaude ou éventuellement froide,
- optionnellement, avant ou éventuellement pendant l'examen lavage mécanique du côlon par une composition aqueuse comprenant de la diméticone ou de la siméticone, de préférence de la diméticone.

Il est également décrit une méthode de préparation à la coloscopie, comprenant les étapes de :
- ingérer, par le patient, la veille au soir de l'examen, en remplacement du dîner :
   ∘ une première composition (B) prise chaude à 60°C et comprenant au moins un légume, notamment comme entrée chaude;
   ∘ une deuxième composition (B) prise chaude à 60°C et comprenant au moins un légume, notamment comme plat chaud;
   ∘ une composition (A) prise à 20°C ou froide comprenant au moins un fruit, de préférence une composition (A2), notamment comme dessert ; et
   ∘ une composition (A) prise chaude à 60°C et comprenant au moins un extrait de plantes ou arôme, de préférence une première composition (A3), notamment comme boisson de préférence chaude ou éventuellement froide,
   ∘ ingérer le jour de l'examen (jour de la coloscopie), de préférence 6 heures avant l'examen, une deuxième composition (A) prise chaude à 60°C et comprenant au moins un extrait de plantes ou arôme, de préférence une deuxième composition (A3), notamment comme boisson de préférence chaude ou éventuellement froide,
- optionnellement, avant ou éventuellement pendant l'examen, lavage mécanique du côlon par une composition aqueuse comprenant de la diméticone ou de la siméticone, de préférence de la diméticone.

### Composition de la solution de lavage pour lavage mécanique :

Flacon monodose à usage unique à diluer dans deux litres d'eau (Réservoir de la flushing pump) :

| |
|---|
| Dimeticone 4 g dissout dans 15cc d'eau stérile. |
| Colorant rouge cochenille. (E 124) |
| Azorubine (E 122) |

### Formation de mousse dans la lumière digestive colique:

Sur une série de vingt patients consécutifs ayant eu une coloscopie après une préparation par macrogols typiquement utilisé actuellement, il a été constaté dans 70% des cas, la formation de mousse lors de la procédure de lavage effectuée durant l'examen. La pompe de lavage était la Flushing pump Olympus OFP2. Le liquide utilisé était de l'eau. La mousse pouvait masquer jusqu'à 90% de la lumière digestive. Le phénomène était observé quelque soit la qualité de la préparation colique. La mousse pouvait se former sur une surface digestive parfaitement propre en l'absence de selles résiduelles. Cette mousse persistait suffisamment longtemps pour perturber significativement l'exploration colique.

Lors d'une coloscopie chez un patient correctement préparé par macrogols, la lumière digestive a été lavée à l'eau à l'aide de la Flushing pump Olympus OFP2 et constaté la formation de mousse masquant 80% de la lumière digestive. Ensuite, au cours de la même procédure, toujours chez le même patient, le liquide de lavage a été remplacé par une solution de Dimeticone 4 g et Colorant (rouge cochenille. (E 124) Azorubine (E 122)) diluée dans deux litres d'eau du réservoir de la flushing pump, soit une concentration de 2 g/l de Diméticone. Avec ce nouveau liquide de lavage la formation de mousse n'est plus constatée.

L'effet anti mousse de ce nouveau liquide de lavage a été confirmé sur une série de dix patients consécutifs préparés par macrogols. Durant la coloscopie, un lavage de la lumière digestive a été effectué à l'aide de la Flushing pump Olympus OFP2 en utilisant une solution de Diméticone 4 g et Colorant E 124 - E 122 diluée dans deux litres d'eau (soit une concentration de 2 g/l de Diméticone). La formation de mousse n'a été constatée chez aucun patient.

### Exemple 1 :

### Introduction de la Diméticone dans la préparation colique absorbée par les patients

Dix patients ont eu une exploration colique avec préparation par macrogols associée à la prise de Diméticone comme décrit ci-dessous. Après lavage à la mécanique, la mousse était totalement absente chez huit patients. Chez deux patients les bulles étaient minimes, ne masquaient jamais la muqueuse et disparaissaient en quelques secondes en raison de l'effet tension actif de la Diméticone.

### Amélioration de la présentation gustative

Une première prise le soir, la veille de l'examen, comporte une entrée chaude à 60°C, un plat chaud à 60°C, un dessert et une boisson chaude de préférence.

La deuxième prise, six heures avant l'exploration endoscopique comporte une boisson prise chaude de préférence à 60°C ou éventuellement froide. Ses qualités gustatives rappellent celle d'une infusion sucrée.

Son impact est fort car le mésusage des préparations coliques existantes (compréhension et la tolérance) est constaté chez un quart des patients (étude observationnelle sur 1000 patients, réalisée à la demande de l'ANSM)

### 1/Prise le soir : Entrée Plat Dessert

Entrée : Fécule de pomme de terre, arômes, graisse de poule, arôme de romarin, légumes 1,1% (oignon, carottes, céleri) épice, aromates, curcuma, jus d'oignon. : (5g)

| **Principes actifs** | **Colomeal soir entrée** |
|---|---|
| Volume de dilution | 250cc |
| Macrogol 3350 | 13,5 g |
| Sulfate de sodium anhydre | 1,3g |
| Chlorure de sodium | 0 ,335g |
| Chlorure de potassium | 0,17g |
| Bicarbonate de sodium | 0,38g |
| Dimeticone | 0,343 g |

Plat : fécule de pomme de terre, légumes 2% (soja, carottes, oignon, poireau, céleri) graisse de poule, arômes blé et soja, arôme de champignons noirs, arôme coco, arôme de romarin. Epices curry, poivre, moutarde, curcuma, coriandre, cannelle. (Total : 33g) Accompagnement : six chips de manioc aromatisées crevettes.

| **Principes actifs** | **Colomeal soir plat** |
|---|---|
| Volume de dilution | 1,0l |
| Macrogol 3350 | 54 g |
| Sulfate de sodium anhydre | 5,2 g |
| Chlorure de sodium | 1,34g |
| Chlorure de potassium | 0,68g |
| Bicarbonate de sodium | 1,54g |
| Dimeticone | 1, 37 g |

Dessert : compote de pomme 70%, banane 30%, cannelle. Stévia, Coulis de framboise. (Total : 120g)

| **Composants** | **Colomeal soir dessert** |
|---|---|
| Volume | 128,90 g |
| Macrogol 3350 | 7,55g |
| Sulfate de sodium anhydre | 0,72 g |
| Chlorure de sodium | 0,19g |
| Chlorure de potassium | 0,1g |
| Bicarbonate de sodium | 0,22g |
| Dimeticone | 0,19g |

Boisson chaude : Stévia. Extraits de plantes 2,4% (menthe, camomille, verveine, oranger, tilleul). Arôme de menthe - Arôme de thé noir (Total : 10g)

| **Composants** | **Colomeal soir boisson** |
|---|---|
| Volume de dilution | 250cc |
| Macrogol 3350 | 13,5 g |
| Sulfate de sodium anhydre | 1,3 g |
| Chlorure de sodium | 0,335g |
| Chlorure de potassium | 0,17g |
| Bicarbonate de sodium | 0,30g |
| Acide Ascorbique | 0,250 g |
| Ascorbate de sodium | 0,250 g |
| Dimeticone | 0,343 g |

### 2/ Prise de liquide H-6 Type infusion

Boisson chaude de préférence à 60°C ou éventuellement froide : Stévia. Extraits de plantes 2,4% (menthe, camomille, verveine, oranger, tilleul). Arôme de menthe Arôme de thé noir. (Total : 60g)

| **Composants** | **Colomeal (h-6) TI°** |
|---|---|
| Volume de dilution | 1,5l |
| Macrogol 3350 | 88,500 g |
| Sulfate de sodium anhydre | 8,5250 g |
| Chlorure de sodium | 2,1990 g |
| Chlorure de potassium | 1,1162 g |
| Bicarbonate de sodium | 1,1g |
| Acide Ascorbique.... | 1 g |
| Ascorbate de sodium | 1 g |
| Dimeticone | 4,4980 g |
| **TI°** type infusion | Boisson type infusion1,5 l |

### Détail des expériences :

20 patients âgés de plus de 75 ans, hospitalisés pour une préparation colique ont été préparés avec les compositions décrites ci-dessus (EPD=Entrée/Plat/Dessert). L'endoscopie était pratiquée entre 11 et 12h30. Les scores de préparation étaient excellents chez tous les patients.

### Horaires de préparation fractionnée (split dose) pour colomeal :

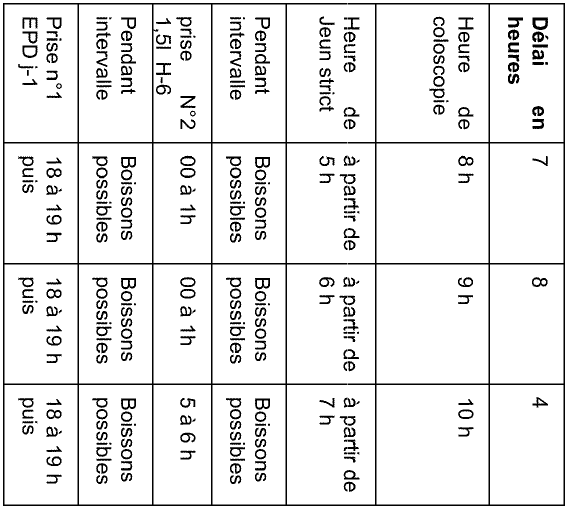

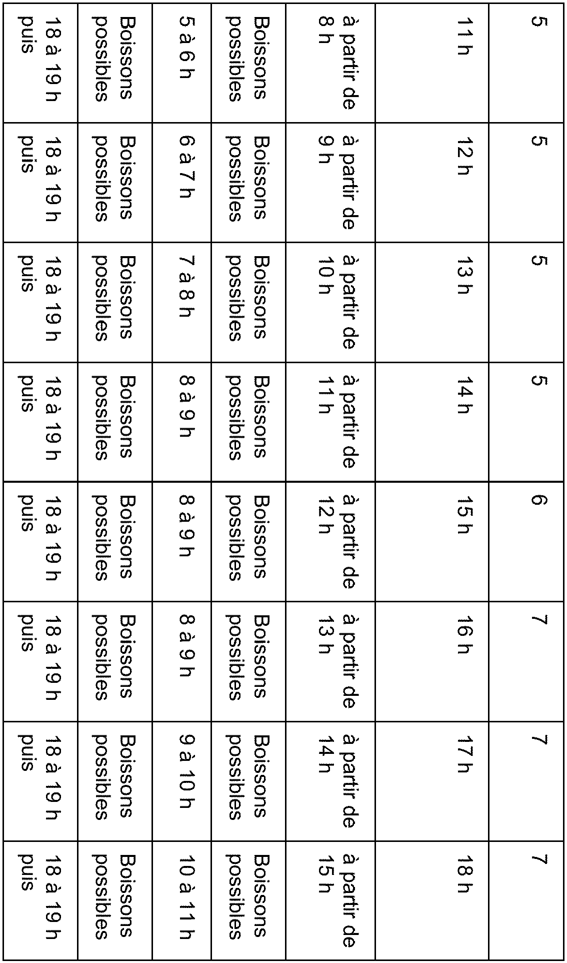

### EPD : Macrogol sous forme d'entrée, plat, dessert.

### J-1 : la veille de la coloscopie

**Macrogol H-6** Type infusion le jour de la coloscopie.

**Pendant intervalle Boissons possibles** : liquides clairs à volonté (thé, café, infusions avec ou sans sucre, eau plate ou gazeuse, jus de fruits : pomme, raisin, orange sans pulpe.)

**Jeun strict** : Plus de boisson, pas de chewing gum, ne pas fumer.

**Délai en heures** : entre la deuxième prise de macrogol H-6 et l'endoscopie

Pour les endoscopies de l'après midi, possibilité de proposer une *alternative* de préparation en un jour, cette modalité est généralement mieux tolérée et jugée plus efficace que la préparation fractionnée (Split dose)

### Horaires de préparation le jour même (préparation alternative)

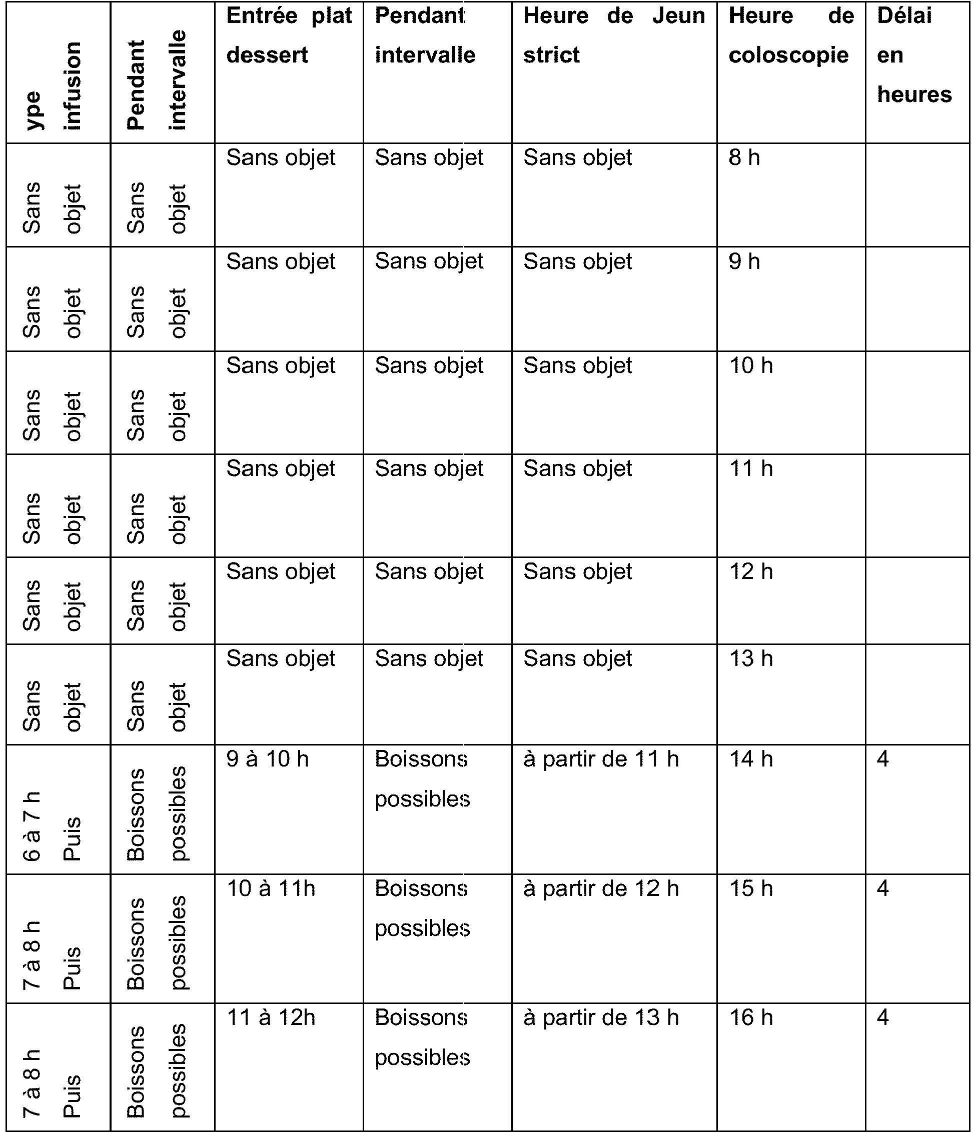

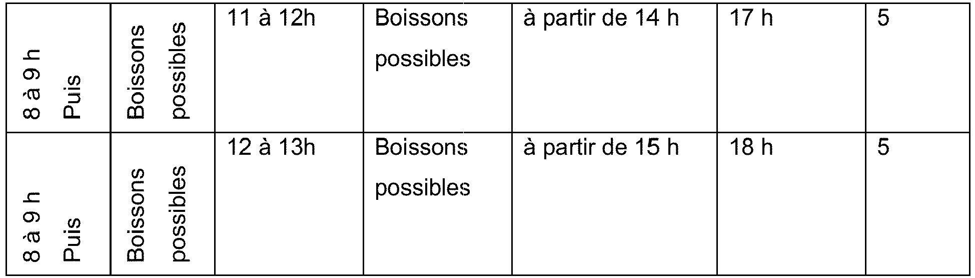

**Préparation Type infusion** : **Macrogol** Type infusion le jour de la coloscopie.

**Pendant intervalle Boissons possibles** : liquides clairs à volonté (thé, café, infusions avec ou sans sucre, eau plate ou gazeuse, jus de fruits : pomme, raisin, orange sans pulpe.)

**EPD : Macrogol sous forme d'entrée, plat, dessert.**

**Jeun strict** : Plus de boisson, pas de chewing gum, ne pas fumer.

**Délai en heures** : entre la deuxième prise de macrogol sous forme d'entrée, plat, dessert et l'endoscopie

### Exemple 2 :

### Introduction de la Diméticone dans la préparation colique absorbée par les patients

Dix patients ont eu une exploration colique avec préparation par macrogols associée à la prise de Diméticone comme décrit ci-dessous. Après lavage à la mécanique, la mousse était totalement absente chez huit patients. Chez deux patients les bulles étaient minimes, ne masquaient jamais la muqueuse et disparaissaient en quelques secondes en raison de l'effet tension actif de la Diméticone.

### Amélioration de la présentation gustative

Une première prise le soir, la veille de l'examen, comporte une entrée chaude à 60°C, un plat chaud à 60°C, un dessert et une boisson chaude de préférence.

La deuxième prise, six heures avant l'exploration endoscopique comporte une boisson prise chaude de préférence à 60°C ou éventuellement froide. Ses qualités gustatives rappellent celle d'une infusion sucrée.

Son impact est fort car le mésusage des préparations coliques existantes (compréhension et la tolérance) est constaté chez un quart des patients (étude observationnelle sur 1000 patients, réalisée à la demande de l'ANSM) 1/Prise le soir : Entrée Plat Dessert

Entrée : Fécule de pomme de terre, arômes, graisse de poule, arôme de romarin, légumes 1,1% (oignon, carottes, céleri) épice, aromates, curcuma, jus d'oignon. : (5g)

| **Principes actifs** | **Colomeal soir entrée** |
|---|---|
| Volume de dilution | 500 cc |
| Macrogol 3350 | 29,5 g |
| Sulfate de sodium anhydre | 2,84 g |
| Chlorure de sodium | 0,73 g |
| Chlorure de potassium | 0,37g |
| Bicarbonate de sodium | 0,84g |

Plat : fécule de pomme de terre, légumes 2% (soja, carottes, oignon, poireau, céleri) graisse de poule, arômes blé et soja, arôme de champignons noirs, arôme coco, arôme de romarin. Epices curry, poivre, moutarde, curcuma, coriandre, cannelle. (Total : 33g) Accompagnement : six chips de manioc aromatisées crevettes.

| **Principes actifs** | **Colomeal soir plat** |
|---|---|
| Volume de dilution | 1000 cc |
| Macrogol 3350 | 59 g |
| Sulfate de sodium anhydre | 5,6 g |
| Chlorure de sodium | 1,46g |
| Chlorure de potassium | 0,74g |
| Bicarbonate de sodium | 1,68g |

Dessert : compote de pomme 70%, banane 30%, cannelle. Coulis de framboise. (Total : 120g)

| **Composants** | **Colomeal soir dessert** |
|---|---|
| Volume | 128,90 g |
| Macrogol 3350 | 7,55g |
| Sulfate de sodium anhydre | 0,72 g |
| Chlorure de sodium | 0,19g |
| Chlorure de potassium | 0,1g |
| Bicarbonate de sodium | 0,22g |
| Dimeticone | 0,19 g |

Boisson chaude : Aspartam 0,095 g. Extraits de plantes 2,4% (menthe, camomille, verveine, oranger, tilleul). Arôme de menthe - Arôme de thé noir (Total : 20g)

| **Composants** | **Colomeal soir boisson** |
|---|---|
| Volume de dilution | 500cc |
| Macrogol 3350 | 29,5 g |
| Sulfate de sodium anhydre | 2,84 g |
| Chlorure de sodium | 0,73g |
| Chlorure de potassium | 0,373g |
| Bicarbonate de sodium | 0,84g |
| Dimeticone | 2,25 g |

### 2/ Prise de liquide H-6 Type infusion

Boisson chaude de préférence à 60°C ou éventuellement froide : Aspartam 0,285 g. Extraits de plantes 2,4% (menthe, camomille, verveine, oranger, tilleul). Arôme de menthe Arôme de thé noir. (Total : 60g)

| **Composants** | **Colomeal (h-6) TI°** |
|---|---|
| Volume de dilution | 1,5l |
| Macrogol 3350 | 88,500 g |
| Sulfate de sodium anhydre | 8,5250 g |
| Chlorure de sodium | 2,1990 g |
| Chlorure de potassium | 1,1162 g |
| Bicarbonate de sodium | 2,52g |
| Dimeticone | 4,4980 g |
| **TI°** type infusion | Boisson type infusion 1,5 l |

### Détail des expériences :

20 patients âgés de plus de 75 ans, hospitalisés pour une préparation colique ont été préparés avec les compositions décrites ci-dessus (EPD=Entrée/Plat/Dessert). L'endoscopie était pratiquée entre 11 et 12h30. Les scores de préparation étaient excellents chez tous les patients.

**Horaires de préparation fractionnée (split dose) pour colomeal :**

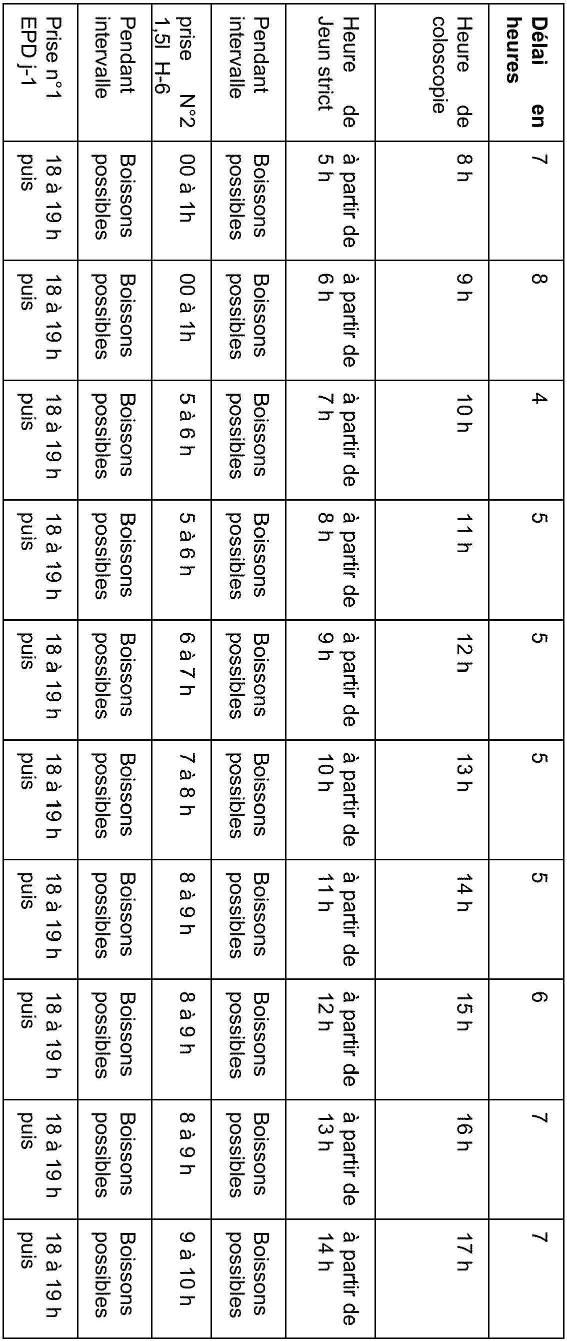

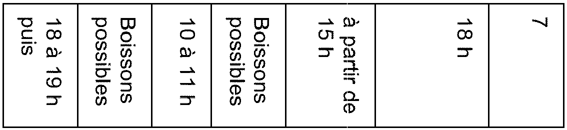

**EPD** : **Macrogol sous forme d'entrée, plat, dessert.**

**J-1** : **la veille de la coloscopie**

**Macrogol H-6** Type infusion le jour de la coloscopie.

**Pendant intervalle Boissons possibles** : liquides clairs à volonté (thé, café, infusions avec ou sans sucre, eau plate ou gazeuse, jus de fruits : pomme, raisin, orange sans pulpe.)

**Jeun strict** : Plus de boisson, pas de chewing gum, ne pas fumer.

**Délai en heures** : entre la deuxième prise de macrogol H-6 et l'endoscopie

Pour les endoscopies de l'après midi, possibilité de proposer une *alternative* de préparation en un jour, cette modalité est généralement mieux tolérée et jugée plus efficace que la préparation fractionnée (Split dose)

### Horaires de préparation le jour même (préparation alternative)

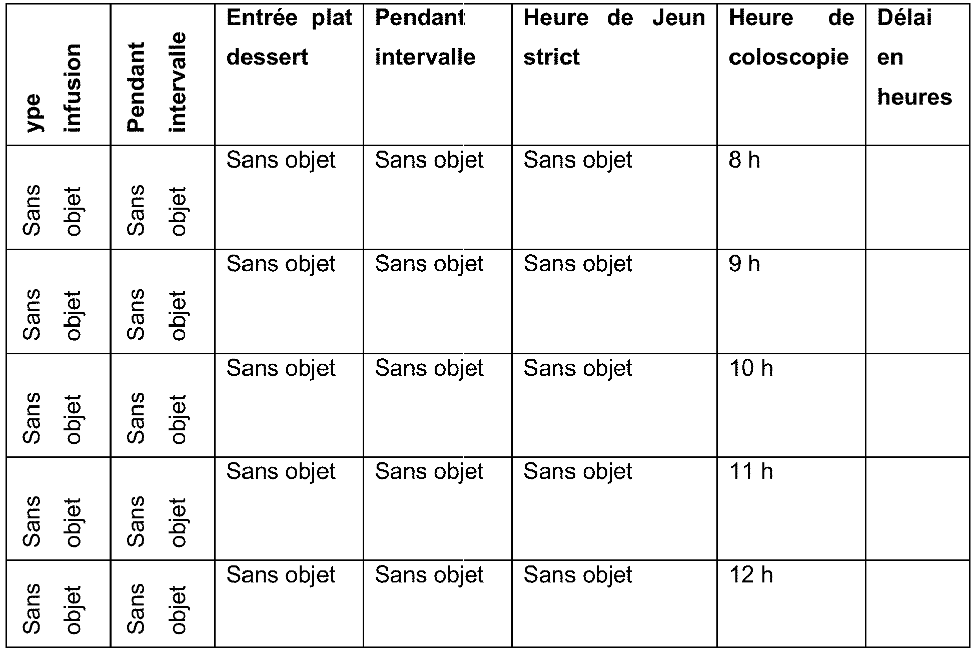

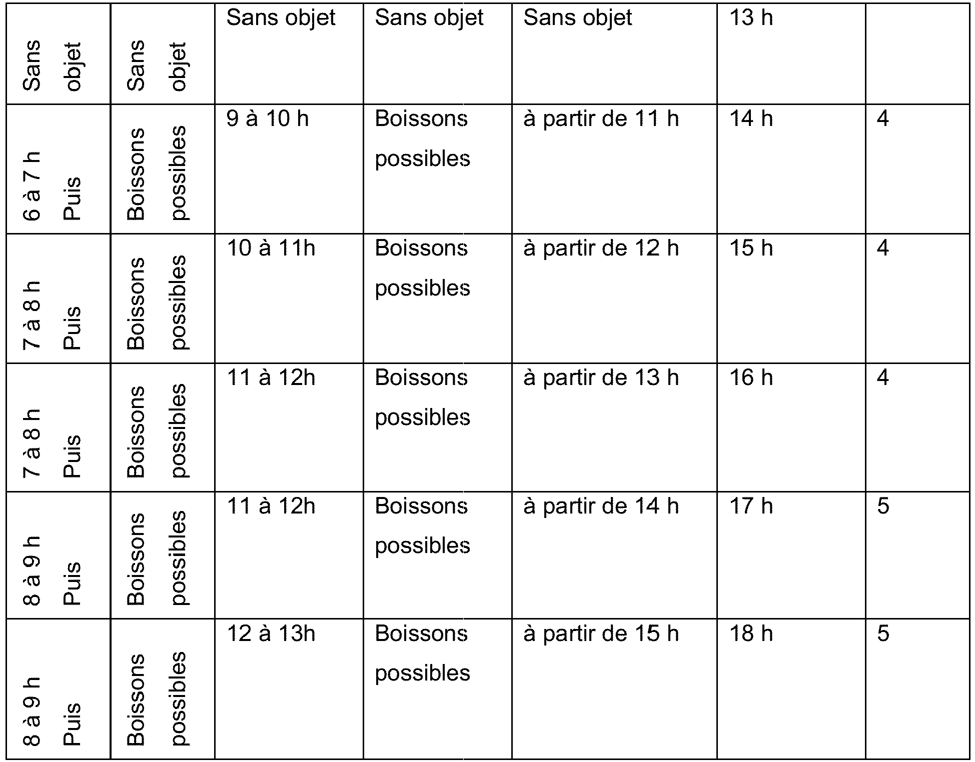

**Préparation Type infusion** : **Macrogol** Type infusion le jour de la coloscopie.

**Pendant intervalle Boissons possibles** : liquides clairs à volonté (thé, café, infusions avec ou sans sucre, eau plate ou gazeuse, jus de fruits : pomme, raisin, orange sans pulpe.)

**EPD** : **Macrogol sous forme d'entrée, plat, dessert.**

**Jeun strict** : Plus de boisson, pas de chewing gum, ne pas fumer.

**Délai en heures** : entre la deuxième prise de macrogol sous forme d'entrée, plat, dessert et l'endoscopie

## Revendications

1. - Kit comprenant :
• au moins une composition déshydratée à reconstituer avec de l'eau et à prendre chaude à 60°C comprenant :
(a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ;
(d) au moins un légume; et
• au moins une composition déshydratée à reconstituer avec de l'eau comprenant :
(a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ;
(d) au moins un fruit, et
• au moins une composition déshydratée à reconstituer avec de l'eau et à prendre chaude à 60°C comprenant :
(a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ;
(d) au moins un extrait de plantes,
pour son utilisation séquencée pour induire une élimination des selles préalable à la coloscopie.

2. - Kit pour son utilisation selon la revendication 1 comprenant :
• deux compositions déshydratées à reconstituer avec de l'eau et à prendre chaudes à 60°C comprenant :
(a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ;
(d) au moins un légume; et
• une composition déshydratée à reconstituer avec de l'eau comprenant :
(a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ;
(d) au moins un fruit, et
• deux compositions deshydratées à reconstituer avec de l'eau et à prendre chaudes à 60°C comprenant :
(a) au moins un composé susceptible de générer des contractions de l'intestin choisi parmi le sulfate de sodium, les picosulfates de sodium, les phosphates de sodium ; les sulfates de magnésium ; les sulfates de potassium ; les polyethylene glycol (PEG), seuls ou en mélange ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone ;
(d) au moins un extrait de plante ou arôme.

3. - Kit pour son utilisation selon la revendication 1 ou 2, dans lequel les compositions comprennent entre 1 et 3% en poids de diméthicone.

4. - Kit pour son utilisation selon l'une des quelconque des revendications 1 à 3, comprenant en outre une composition aqueuse de lavage mécanique du côlon comprenant de la diméthicone.

5. - Kit pour son utilisation selon la revendication 4 dans lequel la composition aqueuse comprend de 0,625 g à 4,5 g, de préférence de 1,125 g à 2,25 g, de diméticone par litre de composition.

6. - Kit pour son utilisation selon l'une quelconque des revendications 1 à 9, dans lequel les compositions sont exemptes de citrate de sodium.

7. Composition déshydratée (A), à reconstituer avec de l'eau et à prendre chaude à 60°C, comprenant :
(a) de 60 à 90 % en poids de PEG ;
(b) au moins un agent osmotique favorisant la rétention d'eau et la pression osmotique dans le côlon, par exemple chlorure de sodium, chlorure de potassium, bicarbonate de sodium, citrate de magnésium, seuls ou en mélange ;
(c) diméticone;
(d) agents de saveurs choisis parmi des légumes, pomme de terre, épices, substitut de sucre, arômes, bouillon de viande (notamment de volaille), extraits de plantes (notamment extrait de menthe, camomille, verveine, oranger et/ou tilleul), seuls ou en mélange,
pour son utilisation pour induire une élimination des selles lors de procédures diagnostique, thérapeutique ou chirurgicales sur l'intestin.

8. Composition déshydratée (A), à reconstituer avec de l'eau et à prendre chaude à 60°C, comprenant un polyéthylène glycol (PEG), du sulfate de sodium, du chlorure de sodium, du chlorure de potassium, du bicarbonate de sodium, de la diméticone et des agents de saveurs choisis parmi des légumes, pomme de terre, épices, substitut de sucre, arômes, bouillon de viande, extraits de plantes, seuls ou en mélange, pour son utilisation pour induire une élimination des selles lors de procédures diagnostique, thérapeutique ou chirurgicales sur l'intestin.

## Patentansprüche

1. Kit, umfassend:
- mindestens eine dehydrierte Zusammensetzung zum Rekonstituieren mit Wasser und zum Einnehmen in warmem Zustand bei 60 ºC, umfassend:
(a) mindestens eine Verbindung, die dazu geeignet ist, Darmkontraktionen hervorzurufen, ausgewählt aus Natriumsulfat, Natriumpicosulfaten, Natriumphosphaten, Magnesiumsulfaten; Kaliumsulfaten; Polyethylenglykolen (PEG), alleine oder in Mischung;
(b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
(c) Dimeticon;
(d) mindestens ein Gemüse; und
- mindestens eine dehydrierte Zusammensetzung zum Rekonstituieren mit Wasser, umfassend:
(a) mindestens eine Verbindung, die dazu geeignet ist, Darmkontraktionen hervorzurufen, ausgewählt aus Natriumsulfat, Natriumpicosulfaten, Natriumphosphaten; Magnesiumsulfaten; Kaliumsulfaten; Polyethylenglykolen (PEG), alleine oder in Mischung;
(b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
(c) Dimeticon;
(d) mindestens eine Frucht, und
mindestens eine dehydrierte Zusammensetzung zum Rekonstituieren mit Wasser und zum Einnehmen in warmem Zustand bei 60 ºC, umfassend:
(a) mindestens eine Verbindung, die dazu geeignet ist, Darmkontraktionen hervorzurufen, ausgewählt aus Natriumsulfat, Natriumpicosulfaten, Natriumphosphaten; Magnesiumsulfaten; Kaliumsulfaten; Polyethylenglykolen (PEG), alleine oder in Mischung;
(b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
(c) Dimeticon;
(d) mindestens ein Pflanzenextrakt für seine sequenzierte Verwendung, um eine Eliminierung des Stuhls von der Koloskopie zu induzieren.

2. Kit für seine Verwendung nach Anspruch 1, umfassend:
- zwei dehydrierte Zusammensetzungen zum Rekonstituieren mit Wasser und zum Einnehmen in warmem Zustand bei 60 ºC, umfassend:
(a) mindestens eine Verbindung, die dazu geeignet ist, Darmkontraktionen hervorzurufen, ausgewählt aus Natriumsulfat, Natriumpicosulfaten, Natriumphosphaten; Magnesiumsulfaten; Kaliumsulfaten; Polyethylenglykolen (PEG), alleine oder in Mischung;
(b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
(c) Dimeticon;
(d) mindestens ein Gemüse; und
eine dehydrierte Zusammensetzung zum Rekonstituieren mit Wasser, umfassend:
( a) mindestens eine Verbindung, die dazu geeignet ist, Darmkontraktionen hervorzurufen, ausgewählt aus Natriumsulfat, Natriumpicosulfaten, Natriumphosphaten; Magnesiumsulfaten; Kaliumsulfaten; Polyethylenglykolen (PEG), alleine oder in Mischung;
( b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
( c) Dimeticon;
( d) mindestens eine Frucht, und
zwei dehydrierte Zusammensetzungen zum Rekonstituieren mit Wasser und zum Einnehmen in warmem Zustand bei 60 ºC, umfassend:
( a) mindestens eine Verbindung, die dazu geeignet ist, Darmkontraktionen hervorzurufen, ausgewählt aus Natriumsulfat, Natriumpicosulfaten, Natriumphosphaten, Magnesiumsulfaten; Kaliumsulfaten; Polyethylenglykolen (PEG), alleine oder in Mischung;
( b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
( c) Dimeticon;
( d) mindestens ein Pflanzenextrakt oder Aroma.

3. Kit für seine Verwendung nach Anspruch 1 oder 2, wobei die Zusammensetzungen zwischen 1 und 3 Gew.% Dimethicon umfassen.

4. Kit für seine Verwendung nach einem der Ansprüche 1 bis 3, umfassend außerdem eine wässrige Zusammensetzung zum mechanischen Waschen des Colons, umfassend Dimethicon.

5. Kit für seine Verwendung nach Anspruch 4, wobei die wässrige Zusammensetzung, von 0,625 g bis 4,5 g, vorzugsweise von 1,125 g bis 2,25 g Dimethicon pro Liter Zusammensetzung umfasst.

6. Kit für seine Verwendung nach einem der Ansprüche 1 bis 9, wobei die Zusammensetzungen frei von Natriumcitrat sind.

7. Dehydrierte Zusammensetzung (A) zum Rekonstituieren mit Wasser und zum Einnehmen in warmem Zustand bei 60 ºC, umfassend:
(a) von 60 bis 90 Gew.% PEG;
(b) mindestens ein osmotisches Mittel, das die Wassereinlagerung und den osmotischen Druck im Colon begünstigt, z. B. Natriumchlorid, Kaliumchlorid, Natriumbicarbonat, Magnesiumcitrat, alleine oder in Mischung;
(c) Dimeticon;
(d) Geschmackstoffe, ausgewählt aus Gemüse, Kartoffeln, Gewürzen, Zuckerersatz, Aromen, Fleischbrühe (insbesondere von Geflügel), Pflanzenextrakten (insbesondere Pfefferminz-, Kamillen-, Eisenkraut-, Orangen- und/oder Lindenextrakt), alleine oder in Mischung, für ihre Verwendung, um eine Elimination des Stuhls bei diagnostischen, therapeutischen oder chirurgischen Verfahren am Darm hervorzurufen.

8. Dehydrierte Zusammensetzung (A) zum Rekonstituieren mit Wasser und zum Einnehmen in warmem Zustand bei 60 ºC, umfassend Polyethylenglycol (PEG), Natriumsulfat, Natriumchlorid, Kaliumchlorid, Natriumbicarbonat, Dimeticon und Geschmackstoffe, ausgewählt aus Gemüse, Kartoffeln, Gewürzen, Zuckerersatz, Aromen, Fleischbrühe, Pflanzenextrakten, alleine oder in Mischung, für ihre Verwendung, um eine Elimination des Stuhls bei diagnostischen, therapeutischen oder chirurgischen Verfahren am Darm hervorzurufen.

## Claims

1. A kit comprising:
• at least one dehydrated composition to be reconstituted with water and to be taken hot at 60 °C comprising:
(a) at least one compound able to generate contractions of the intestine, selected from among sodium sulfate, sodium picosulfates, sodium phosphates; magnesium sulfates; potassium sulfates; polyethylene glycol (PEG), alone or in a mixture;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) at least one vegetable; and
• at least one dehydrated composition to be reconstituted with water comprising:
(a) at least one compound able to generate contractions of the intestine, selected from among sodium sulfate, sodium picosulfates, sodium phosphates; magnesium sulfates; potassium sulfates; polyethylene glycol (PEG), alone or in a mixture;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) at least one fruit; and
• at least one dehydrated composition to be reconstituted with water and to be taken hot at 60 °C comprising:
(a) at least one compound able to generate contractions of the intestine, selected from among sodium sulfate, sodium picosulfates, sodium phosphates; magnesium sulfates; potassium sulfates; polyethylene glycol (PEG), alone or in a mixture;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) at least one plant extract;
for sequential use thereof to induce elimination of stools prior to colonoscopy.

2. A kit for use thereof according to claim 1, comprising:
• two dehydrated compositions to be reconstituted with water and to be taken hot at 60 °C comprising:
(a) at least one compound able to generate contractions of the intestine, selected from among sodium sulfate, sodium picosulfates, sodium phosphates; magnesium sulfates; potassium sulfates; polyethylene glycol (PEG), alone or in a mixture;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) at least one vegetable; and
• a dehydrated composition to be reconstituted with water comprising:
(a) at least one compound able to generate contractions of the intestine, selected from among sodium sulfate, sodium picosulfates, sodium phosphates; magnesium sulfates; potassium sulfates; polyethylene glycol (PEG), alone or in a mixture;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) at least one fruit; and
• two dehydrated compositions to be reconstituted with water and to be taken hot at 60 °C comprising:
(a) at least one compound able to generate contractions of the intestine, selected from among sodium sulfate, sodium picosulfates, sodium phosphates; magnesium sulfates; potassium sulfates; polyethylene glycol (PEG), alone or in a mixture;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) at least one plant extract or flavouring;

3. The kit for use thereof according to claim 1 or 2, wherein the compositions comprise from 1 to 3 weight % dimethicone.

4. The kit for use thereof according to any of claims 1 to 3, further comprising an aqueous composition comprising dimethicone for mechanical cleansing of the colon.

5. The kit for use thereof according to any of claims 1 to 4, wherein the aqueous composition comprises from 0.625 to 4.5 g, preferably 1.125 to 2.25 g of dimethicone per litre of composition.

6. The kit for use thereof according to any of claims 1 to 9, wherein the compositions are free of sodium citrate.

7. A dehydrated composition (A) to be reconstituted with water and taken hot at 60 °C, comprising:
(a) from 60 to 90 weight % PEG;
(b) at least one osmotic agent promoting water retention and osmotic pressure in the colon e.g. sodium chloride, potassium chloride, sodium bicarbonate, magnesium citrate, alone or in a mixture;
(c) dimeticone;
(d) taste enhancers selected from among vegetables, potato, spices, sugar substitute, flavourings, meat stock (in particular poultry stock), plant extracts (in particular extracts of mint, camomile, verbena, orange blossom and/or lime blossom) alone or in a mixture for use thereof to induce elimination of stools for diagnostic, therapeutic or surgical intestinal procedures.

8. A dehydrated composition (A) to be reconstituted with water and to be taken hot at 60°C, comprising a polyethylene glycol (PEG), sodium sulfate, sodium chloride, potassium chloride, sodium bicarbonate, dimethicone and taste enhancers selected from among vegetables, potato, spices, sugar substitute, flavourings, meat stock, plant extracts alone or in a mixture, for use thereof to induce elimination of stools for diagnostic, therapeutic or surgical intestinal procedures.
